# EUROPEAN PATENT APPLICATION

(11) **EP 3 954 379 A1**
(43) Date of publication of application: **16.02.2022**
(21) Application number: 20190732.6
(22) Date of filing: 12.08.2020
(51) Int. Cl.: A61K 36/47, A61K 9/00, A61P 31/00

(54) **COMPOSITIONS COMPRISING PHYLLANTHUS EXTRACT FOR USE IN THE TREATMENT OR PREVENTION OF A SARS-COV-2 INFECTION AND/OR AT LEAST ONE SYMPTOM OF COVID-19**

(71) Applicant: HFM - Hybrid Fusion Medicals, GmbH, 82515 Wolfratshausen (DE)
(72) Inventor: VON KEUDELL, Christoph., 82515 Wolfratshausen (DE)

(57) **Abstract**

The present invention relates to a composition comprising or corresponding to a *Phyllanthus* extract for use in the treatment or prevention of a SARS-CoV-2 infection and/or at least one symptom of COVID-19.

## Description

The present invention relates to a composition comprising or corresponding to a *Phyllanthus* extract for use in the treatment or prevention of a severe acute respiratory syndrome coronavirus-2 (SARS-CoV-2) infection and/or at least one symptom of coronavirus disease-19 (COVID-19).

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Betacoronaviruses (β-CoVs or Beta-CoVs) are one of four genera of coronaviruses of the subfamily *Orthocoronavirinae* in the family *Coronaviridae*, of the order *Nidovirales.* They are enveloped, positive-sense, single-stranded RNA viruses of mostly zoonotic origin. The genome of betacoronaviruses encodes four structural proteins, known as the S (spike), E (envelope), M (membrane), and N (nucleocapsid) proteins. The N protein holds the RNA genome, and the S, E, and M proteins together create the viral envelope. The spike protein is the major glycoprotein on the coronavirus surface and is responsible for allowing the virus to attach to and fuse with the membrane of a host cell. The spike protein forms a crown-like structure on the surface of a coronaviruses.

COVID-19, a contagious disease caused by the novel coronavirus SARS-CoV-2 and first observed in Wuhan, China in late 2019, has developed into a pandemic having affected more than 10.4 Mio. people worldwide and having caused more than 509.000 deaths by the end of June 2020. An enormous effort involving a large number of companies and research institutes is underway to develop vaccines for controlling the disease and therapeutic agents for treating patients that suffer from symptoms of COVID-19. Despite some promising initial results, no vaccine will be available for at least the next few months that would meet the need of providing protection of millions of people. Similarly, some medicaments like Remdesivir seem to have some curative effects but patients treated with the presently available more or less promising medicaments are expected to suffer from sometimes serious side effects. Consequently, there remains a need for well tolerated therapeutic agents that are effective in the prevention and/or treatment of COVID-19 and secondary symptoms caused by this disease. This need is addressed by the present invention.

Accordingly, the present invention relates to a composition comprising or corresponding to a *Phyllanthus* extract for use in the treatment or prevention of a SARS-CoV-2 infection and/or at least one symptom (also referred to herein as "disease manifestation" or "manifestation") of COVID-19.

The present invention also relates to a method for the treatment or prevention of a SARS-CoV-2 infection and/or at least one symptom of COVID-19 comprising administering to a subject a therapeutically effective amount of a composition comprising or corresponding to a *Phyllanthus* extract. In this respect, the subject is in general a human.

The term "*Phyllanthus*" in accordance with this invention has an art-established meaning. It thus refers to the various species of this genus. There are some 700 species of *Phyllanthus* organized in ten subgenera (Holm Nielsen LB. Comments on the Distributions and Evolution of the Genus Phyllanthus (Euphorniaceae). In: Larseno K, editor. Tropical Botany, Academic Press. 1979: 277-290). The longstanding interest in *Phyllanthus* is based on its historical use in herbal medicine for a variety of conditions, most commonly for the treatment of jaundice. A number of species of the genus *Phyllanthus* (Euphorbiaceae) have been tested for their efficacy as antivirals, partly on the basis of references to traditional usage for the treatment of diseases possibly having a viral origin. Herbs of the subgenus *Phyllanthus phyllanthus* have been most extensively used as traditional medicines, including nine species with recorded use to treat jaundice (Unander DW, Webster GL, Blumberg BS. Usage and bioassays in Phyllanthus (Euphorbiaceae) - IV: Clustering of antiviral uses and other effects. J Ethnopharmacol 1995; 45(1):1-18); one of these was *Phyllanthus amarus.* A further *Phyllanthus* subspecies that has only been observed in the Americas and that has been used in various therapeutic settings is *Phyllanthus niuri.* In particular for extracts of *Phyllanthus amarus* some positive results in the treatment of Hepatitis B infections have been reported (e.g. Thyagarajan SP et al., Effect of Phyllanthus amarus on chronic carriers of hepatitis B virus. Lancet 1988; 2(8614):764-766).

Plants of the genus *Phyllanthus* contain secondary plant ingredients such as gallotannins including corilagin, geraniin, brevifolin carboxylic acid or ellagitannins. *Phyllanthus* extracts, *inter alia,* also can comprise proteins, sterols, lignans, flavonol-glycosides, polysaccharides and alkaloids. Some of those components, under the influence of light, temperature, oxygen and heavy-metals, are subject to oxidative degradation processes. In this respect, hydrolysable tanning agents are to be mentioned, such as the didehydrohexahydroxydiphenol amariine or geraniine, a ellagitannin dominating in quantity. An additional group of ingredients (lignanes), the phyllanthines, such as present in *Phyllanthus amarus* Schumach. et Thonn., whereby, primarily, phyllanthine and hypophyllanthine are to be mentioned. Phyllanthines and ellagitannins have in common their antioxidative reactivity, which characterises them, on the one hand, as important active agents and, on the other hand, as easily destructible. Whereas the ellagitannins are substances which are polar regarding their physico-chemistry and are very easily water-soluble, the phyllanthines preferably dissolve in organic solvents or mixtures of the latter with water. Both substance classes occur, alongside a series of ubiquitous primary and secondary plant ingredients, in aqueous preparations (Infuse/Decocte), as common in traditional folk medicine. With regard to the solving behavior of individual components, complex mixtures of several substances thus behave completely different, as the case may be, than would be expected for one ingredient alone. Both for the drug *Phyllanthus amarus* itself and for aqueous and alcoholic preparations (methanol, ethanol, butanol), there is a series of contradicting pharmacological data *in vitro* and *in vivo.* In particular, results of analyses regarding an antiviral activity range from strongly effective to non-effective. For a broad overview see Hager's Handbuch der pharmazeutischen Praxis, 5.sup.th edition, Vol. 3, Drogen L-Z, Springer Verlag, 342 343.

The term "comprising or corresponding to a *Phyllanthus* extract" is intended to mean that in the first case ("comprising") the extract may be further modified, for example, by introducing stabilizing agents into the protein fraction, or may contain additional therapeutically active agents that in one alternative have an anti-viral effect. In the second alternative ("corresponding") the extract is administered without any further modification and without the addition of further therapeutically active compounds. However, fillers or carriers may still be present.

The term "extract" or "plant extract" means any substance or derivative product that is obtained by extraction from one or more parts of the *Phyllanthus* plant(s), by any method known in the state of the art, such as solvent extraction, adsorption, maceration, distillation, among others. The "extract" in accordance with the present invention may be obtained by any extraction method that releases therapeutically active agents, such as agents having anti-viral activity, immunomodulatory activity, anti-fibrotic activity, anti-phlogistic activity, anti-oxidative activity, anti-allergic or antimicrobial activity. Preferably, the extract is an alcohol-water based extract such as an ethanol-water extract, a methanol-water extract or a butanol-water extract. Particularly preferred is an ethanol-water extract. Examples of such extracts are described in more detail herein below.

The extract can be obtained from all the constituents of the whole plants such as e.g. leaves, bark, blossoms, seeds, fruits, stalks, branches, stems, roots and wood, as well as parts thereof such as e.g. leaf or root apexes. Different *Phyllanthus* constituents can be used individually or together and different *Phyllanthus* constituents of different *Phyllanthus* species can be used individually or together. "Several" *Phyllanthus* constituents relates to some or all of the *Phyllanthus* constituents. The *Phyllanthus* extracts can be used after pre-treatment or without pre-treatment. Pre-treatment could, for example, comprise procedures such as the drying of leaves; see below.

The constituents of a *Phyllanthus* species ethanol-water extract of the leaves ("PTX-111 native extract"), for example specifically from leaves of *Phyllanthus amarus* Schumach. et Thonn., obtained/obtainable e.g. by conducting step (a) of the herein disclosed method, as known of today are as follows:

### COMPOSITION OF PTX-111 NATIVE EXTRACT:

As of to date about 80 to 90 per cent of the *PTX-111* native extract from *Phyllanthus amarus* Schumach. et Thonn. leaves have been already chemically identified.

The Inorganic fraction, determined as ash, mainly consists of Potassium, Calcium, Sodium and Magnesium. Main anion is Chloride. Trace elements Phosphor, Sulphur.

The Lipophilic fraction contains compounds of unsaponifical matter like Phytosterols.

The polar and semipolar fraction contains
- Lignanes (Phyllanthine, Hypophyllanthine and others),
- Tanning agents including Gallotannines, e.g. Geraniin, Corilagin, Ellagic acid as well as a further main Gallotannin compound.
- Proteins
- Free Amino acids
- Polysaccharides
- Flavonoids (Rutosid identified)
- Phenolic compounds
- water
Chlorophyll is just a trace compound (< 0.1 per cent).

### Remarks:

(1): *All test procedures are validated; they refer to the state of scientific experience, related guidelines and pharmacopoes.*

Prior to the extraction, the following measures are typically performed: According to GAP rules quality maintenance measures are applied to the harvested plant material. E. g. maximum time between harvest and drying, drying temperature, and humidity after drying are determined. After drying the plant material, which is in accordance with preferred embodiments the leaves, the leaves and seeds are separated from the stems, packed in paper bags or aerated plastic bags. Every batch of the leaves is evaluated according to HFM Monograph Version 3.0 including TLC and HPLC. Furthermore a characteristic DNA fingerprint of *Phyllanthus,* preferably *Phyllanthus amarus* leaves is performed regularly to avoid mutations and contaminaton with other subspecies. Microbial contamination, pesticide residues, heavy metal residues, aflatoxins and fumigate agents are subject to QC and have to fulfill the specifications.

Within the meaning of the present invention, "prevention" relates to the use of compositions comprising or corresponding to a *Phyllanthus* extract as a prophylaxis for the prevention of the infection with SARS-CoV-2. Furthermore, according to the invention, this relates to avoiding the breakout of the disease or achieving a less severe breakout of the disease. The *Phyllanthus* compositions comprising or corresponding to a *Phyllanthus* extract used for prevention can be different from or identical to the *Phyllanthus* compositions comprising or corresponding to a *Phyllanthus* extract used for treatment. The use for prevention can take place in a different or the same dose and by a different or the same application as in the use for treatment. Prevention can take place by a one-off or by several application(s). The dose of *Phyllanthus* compositions comprising or corresponding to a *Phyllanthus* extract used for prevention should, however, essentially offer sufficient protection against SARS-CoV-2 infection. At the same time, the dose used for prevention should not have a harming effect on the organism, of course. The same holds true for the inventive compositions used in the "treatment" of the COVID-19 or symptoms associated with COVID-19. For the initiation of treatment of COVID-19, symptoms of the disease must be visible. Treatment should then stop or reduce the development or progression of disease symptoms in which these viruses play a role, leading to an at least partial and preferably complete recovery of the affected patients.

It is well known that COVID-19 does not display the same symptoms in every patient and that in different patients different organs may be affected. In that regard, the term "at least one symptom of COVID-19" is intended to refer to disease symptoms that are observed in different organs of affected patients. In so far, the composition for use in accordance with the present invention can successfully be applied to different subgroups of patients.

For the treatment, the extract may be formulated as a pharmaceutical composition, a medicinal device or a food supplement.

In accordance with the present invention, the pharmaceutical composition comprises a herbal medicinal product comprising or corresponding to a *Phyllanthus* extract.

Preferably, the herbal medicinal product is in accordance with the standards set out in the EMA Guideline on declaration of herbal substances and herbal preparations in herbal medicinal products/traditional herbal medicinal products (EMA/HMPC/CVMP/287539/2005ref1).

In accordance with the present invention, the pharmaceutical composition comprises a medicinal product comprising or corresponding to a *Phyllanthus* extract. Preferably, the medicinal product is in accordance with the standards set out in the German MPG paragraph 3 and 93/42EWG.

*Phyllanthus* (also referred to herein as "*Phyllanthus spec.*") may, for example, be selected from the group of *Phyllanthus phyllanthus*, *Phyllanthus amarus*, *Phyllanthus niruri, Phyllanthus emblica*, *Phyllanthus urinaria*, *Phyllanthus myrtifolius*, *Phyllanthus maderas pratensis* and/or *Phyllanthus ussuriensis.*

In a preferred embodiment of the composition of the invention, the *Phyllanthus* extract is an extract of *Phyllanthus amarus* or *Phyllanthus niuri.* In a more preferred embodiment, the *Phyllanthus* is *Phyllanthus amarus.* In a particularly preferred embodiment, the *Phyllanthus amarus* is the *Phyllanthus amarus* Schumach. et Thonn.. As mentioned herein above, in the art there is ample evidence that extracts from these species do not cause any severe side effects. In addition, and as also mentioned herein above, certain varieties of *Phyllanthus amarus* such as *Phyllanthus amarus* Schumach. et Thonn. contain ingredients that are of particular therapeutic value.

In a further preferred embodiment of the composition of the invention, the *Phyllanthus* extract is obtained or obtainable by a method comprising the following step(s):
(a) extracting *Phyllanthus* components with an ethanol/water mixture of 35-45% mass/mass which contains a heavy metal chelator in a concentration of 0.1-1% mass/mass; and optionally
(b) treating and concentrating the extract obtained in (a) with
   (ba) Indian sterculia gum in a final concentration of 0.5 to 5.0%, preferably 0.7 to 1.3%, relative to the overall amount of extracted compounds; and/or
      8.0 - 39% Maltodextrin; and/or
      0.5 - 3.0% Silica, colloidalis anhydrous; and/or
      1.0 - 3.0% Sodium acid citrate, mass/mass; and/or
   (bb) one or more pharmaceutically acceptable polysaccharides in a final concentration of 2-10% mass/mass, relative to the overall amount of extracted compounds; and/or optionally
(c) drying the extract obtained in step (a) or (b).

As used herein, the extract obtained/obtainable by aqueous-alcoholic extraction (e.g. step (a) of the above-described method) is termed "native extract", whereas the extract obtained/obtainable upon conducting in addition a drying (i.e. step (c)) is termed "dry extract".

In a further preferred embodiment, all excipients, more preferable, all raw materials including excipients and plant material, used for preparing the compositions of the invention are in pharmacopoeia quality.

According to a particular preferred embodiment, the composition may be a direct product of the above recited method. On the other hand and since the method has been published (see below), the skilled person can now prepare extracts comprising the therapeutically effective agents by modifying the above method without inventive skills. Therefore, the invention also covers embodiments where the composition is obtainable but not necessarily directly obtained by applying the above recited method steps, i.e. the composition comprises essentially the same compounds in essentially the same concentrations as the composition comprising the compounds directly obtained with the above-recited method. As the drying step is optional, the extract may be formulated into / used as a composition in accordance with the present invention also in liquid form (which may, *inter alia*, be delivered in vaporized form (nebulization) for example for oral or nasal administration by inhalation).

A method that may be applied according to the invention has been described in WO 2002/030436 and leads to particularly high yield of pharmaceutically effective plant ingredients. (The disclosure content of the international application is herewith incorporated in its entirety.) It is thus of particular interest for therapeutic applications. The extract obtainable e.g. by the method obtained in WO 2002/030436 has been termed by the present inventor *PTX-111.*

*PTX-111* thus is a standardized ethanolic extract preparation of the leaves of *Phyllanthus spec.*, preferably from *Phyllanthus amarus* Schumach. et Thonn.. Chemically, PTX-111 is a complex mixture of organic and inorganic compounds. The organic compounds are mainly the pharmacological active substances ranging from hydrophilic characters to lipophilic compounds. As outlined herein above in general terms of *Phyllanthus* plants, these compounds in the *PTX-111* extract are among others also lignans, gallotannins, flavonoids, proteins, free amino acids and polysaccharides. This mixture of compounds exhibits different pharmacological activities which have been demonstrated in preclinical studies with *PTX-111* initiated by the present inventor, albeit in disease models unrelated to COVID-19. These studies have, *inter alia*, shown anti-oxidative, immunomodulatory, anti-fibrotic and anti-inflammatory properties of *PTX-111.*

The anti-viral properties of *PTX-111* and its fractions have been evaluated in HBV and HIV cellular replication models and enzyme assays *in vitro.* Although in various assays reverse transcriptase, polymerase, integrase and protease inhibitory activity has been demonstrated at various, mostly at higher dose levels, its most potent mode of action in cellular models appears to be early pre-RT entry inhibition in HIV.

Certain fractions of and actual active moieties contained in *PTX-111* have been identified to potently exert these activities *in vitro* and the active ingredients of these effects have been identified, but are not publically available. Furthermore, synergistic effects of single compounds and/or their metabolites *in vivo* are expected. Analytical studies to this extent are ongoing.

To date about 80-90 % of the ingredients of the native extract are identified. Also to date, the entire extract is regarded to be the active substance (API), and not considered a combination product.

In one typical approach of preparing the extract, approximately 3.5 - 7.5 g crude plant material is used to yield 1 g of native extract after ethanol/water extraction and subsequent drying under addition of defined technical excipients as disclosed herein above. Following extraction, the concentration of lignans and gallotannins as marker substances during quality measurements are adjusted by blending. The active substance, *PTX-111*, in one embodiment, is a brown powder having a green tinge with a characteristic bitter taste and a characteristic odor.

In an alternative preferred embodiment, the extract comprises the compounds comprised in the *PTX-111* native extract (described herein above), more preferable the extract comprises one or more compounds of the above-defined lipophilic fraction and/or polar and semipolar fraction.

As already indicated herein above, in a further preferred embodiment of the composition of the invention, the *Phyllanthus* components are leaves. Leaves are preferred since the extract *PTX-111* that is used for the therapeutic applications in the treatment or prevention of COVID-19 or downstream symptoms (i.e. secondary symptoms, including extra-pulmonary symptoms as defined herein below) is preferably prepared from leaves.

In a further preferred embodiment of the composition of the invention, after step (a) and prior to step (b) a filtration with the extract obtained in step (a) is carried out, wherein the filter preferably has an exclusion volume of 0.05 - 0.5 µm, more preferable 0.1 - 0.3 µm. The filtration step is preferably an ultrafiltration.

In a further preferred embodiment of the composition of the invention, in step (a) or prior to step (b) a lipid, preferably selected from the group of plant oils, waxes and fatty acids is added in a final concentration of 1 - 500%, preferably 1 - 100% mass/mass, relative to the overall amount of extracted compounds.

Also preferred is that the heavy metal chelator is a multibase organic acid or a salt thereof and particularly preferred is disodium hydrogen citrate.

In a further preferred embodiment of the composition of the invention, the composition is a pharmaceutical composition or a medicinal device. In particular, in the preparation of the pharmaceutical composition, pharmaceutically acceptable carriers or excipients may be added, such as prior to or during the optional drying step. Preferred excipients include maltodextrin and silica, preferably highly dispersed.

Drying is preferably effected by freeze drying, band drying or spray drying.

As mentioned, in one preferred embodiment, the composition is a pharmaceutical composition. In accordance with the present invention, the term "pharmaceutical composition" relates to a composition for administration to a subject, preferably a human subject. The pharmaceutical composition of the invention comprises the compounds recited above. It may, optionally, comprise further molecules capable of altering the characteristics of the compounds of the invention thereby, for example, stabilizing, modulating and/or activating its function. The composition may be in solid, liquid or gaseous form and may be, *inter alia*, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the dose of the extract in the pharmaceutical composition should be in the range of 1000 - 2000 mg of the extract, preferably of the native extract,per administration, preferably 1500 - 2000 mg. Preferably doses in this range should be administered per day (such as in coated tablets comprising 300 mg of extract), but schemes can be changed according to the attending physician's discretion such as twice or three to five times a day (or even more often) or every other day, every three days or every other week. Suitable administration regimens can readily be determined by the ordinary clinician or physician.

The length of treatment needed to observe changes and the interval following treatment for responses to occur vary depending on the progress in the health condition of the COVID-19 patient. The particular amounts may be adjusted by conventional tests for B and T cell immune responses, which are well known to the person skilled in the art. Preliminary data have shown that the extract can be administered over extended periods of time such as over several months without causing any severe side effects.

Administration of the pharmaceutical composition of the invention is preferably by the oral or intravenous route in an alternative preferred embodiment, also depending on the actual symptoms and the disease state of the patient. Also preferred is that the composition comprising or corresponding to the extract is administered as an aerosol (e.g. for the upper and lower respiratory tract), or as a tablet or capsule.

It is of note that the composition to be used in accordance with the present invention acts systemically and is thus not limited to preventing symptoms affecting particular organs of affected patients. Rather, the composition is expected to have an effect on most or all affected organs. Nevertheless, it is also expected that certain compositions enriched for specific *Phyllanthus* derived compounds that can be isolated/purified from natural sources or can be produced synthetically will have beneficial effects on specific affected organs. It is hence also particularly envisaged in certain embodiments that the composition is depleted for certain particular compounds or compound classes in order to remove or substantially reduce one or more particular effects from which a certain subject, due to its particular health condition or pre-disposition, would not benefit from or have a risk of having a complication effected by such an effect. Compositions may for example be depleted for such compounds having immune-activating/-stimulatory activity, if intended for administration to a subject that has an overly-active or hyperactive immune system, for example, a subject already suffering from an autoimmune disease, such as without limitation, rheumatic arthritis or other acute or chronic inflammatory condition. Alternatively or additionally, the compositions can be enriched for such compounds from which effects an individual subject would be expected to benefit from. For example, for an intended administration to a subject with overly active immune system, the composition may be enriched for compounds having anti-inflammatory effects. On the other hand, a composition intended for administration to an immunocompromised subject may be enriched for immune-activating/-stimulatory compounds. It is thereby understood that the compounds for which certain compositions can be enriched may be isolated/enriched from natural sources (i.e. extracts from *Phyllanthus* plants) or originate from synthetic routes. Methods for separating, enriching or depleting individual compounds or groups of compounds from a complex mixture of different compounds are known in the art and can routinely be employed by the skilled person.

Some embodiments dealing with such combinations will be described in the following in more detail. It is also to be understood that certain *Phyllanthus* derived compounds, may they be isolated/purified/enriched from natural sources or be obtained by known synthetic routes, can be modified for changing their pharmacokinetic and/or pharmacodynamic properties, e.g. for achieving an increase in serum half-life and/or a resistance to degradation, for example, by pegylation, glycosylation, or conjugation with albumin (e.g., human serum albumin (HSA)), or by other approaches known in the art for achieving such effects.

The composition for use in accordance with the invention is suitable for administration to all stages of COVID-19, i.e. from the occurrence of initial symptoms until severe grades of the disease develop as well as symptoms such as difficulty of breathing that are observed in some patients after they are considered recovered from COVID-19, in addition to its usefulness in the prevention of the disease. For preventive purposes, oral administration is preferred.

The first step in infection is virus binding to a host cell through its target receptor. Earlier work on SARS-CoV demonstrated that this virus principally targets airway epithelial cells, alveolar epithelial cells, vascular endothelial cells and macrophages in the lung, all of which express the angiotensin-converting enzyme 2 (ACE2) which is known to serve as host target receptor for SARS-CoV, the pathogen responsible for the SARS epidemic of 2003. As SARS-CoV-2 uses the same entry receptor, these cell subsets are likely targeted by this virus. SARS-CoV infection reduces ACE2 expression in lung cells. Because loss of pulmonary ACE2 function is associated with acute lung injury, virus-induced ACE2 downregulation is thought to critically contribute to disease pathology. Specifically, ACE2 has been shown to regulate the renin-angiotensin-aldosterone system (RAAS). A reduction in ACE2 function after viral infection could therefore result in a dysfunction of the RAAS, which influences blood pressure and fluid/electrolyte balance, and enhance inflammation and vascular permeability in the airways (Tay MZ et al., Nat Rev Immunol. 2020;20(6):363-374. "The trinity of COVID-19: immunity, inflammation and intervention" PMID: 32346093).

While SARS-CoV-2 is primarily known to cause substantial pulmonary disease, including pneumonia and acute respiratory distress syndrome (ARDS), many extra-pulmonary manifestations of COVID-19 have also been observed. In particular, evidence has been found that various organs systems beyond the respiratory tract including the hematologic, cardiovascular, renal, gastrointestinal and hepatobiliary, endocrinologic, neurologic, ophthalmologic, and dermatologic systems can be affected (see e.g. Review by Gupta A et al., Nat Med. 2020;26(7):1017-1032. PMID: 32651579; Cha MH et al.; World J Gastroenterol. 2020; 26(19): 2323-2332. PMID: 32476796).

Key mechanisms underlying the pathophysiology of multi-organ injury secondary to infection with SARS-CoV-2 include direct viral toxicity, endothelial cell damage and thromboinflammation, dysregulation of the immune response, and dysregulation of the renin-angiotensin-aldosterone system (RAAS). While some of these mechanisms, including ACE2-mediated viral entry and tissue damage, and dysregulation of the RAAS, may be unique to COVID-19, the immune pathogenesis caused by the systemic release of cytokines and the microcirculation dysfunctions may also occur secondary to sepsis (Gupta A et al.; Nat Med. 2020 Jul;26(7):1017-1032).

Thus, in accordance with a preferred embodiment of the invention, the at least one symptom of COVID-19 is a manifestation of the respiratory system, preferably of the upper and/or lower respiratory tract, and/or manifestation(s) of the lung. Manifestations of the lung include, without limitation, pneumonia, severe pneumonia, pulmonary fibrosis, (acute) lung injury and acute respiratory syndromes, including severe acute respiratory syndrome (SARS) and acute respiratory distress syndrome (ARDS).

Particular reported extra-pulmonary manifestations of COVID-19 which are specifically envisaged for the medical applications of the composition of the present invention are described in the following:
Hematologic and/or immune system-related manifestations of COVID-19 include various forms of hematological abnormalities, including lymphopenia (a.k.a. lymphocytopenia), leukocytosis, leukopenia, neutrophilia, abnormal blood clotting, dysregulated blood coagulation, thrombocytopenia, pulmonary embolism, disseminated intravascular coagulation, deep vein thrombosis, and prothrombotic state.

Cardiovascular manifestations of COVID-19 include myocardial injury, acute cardiac injury, acute coronary syndromes (ACS), cardiomyopathy, acute cor pulmonale, cardia arrhythmias (including new-onset atrial fibrillation, heart attack, heart block, and ventricular arrhythmias), cardiogenic shock, myocardial ischemia, acute cor pulmonale, and/or thrombotic complications.

Renal manifestations of COVID-19 include acute kidney injury (AKI), proteinuria and hematuria; and may be characterized by electrolyte abnormalities (such as hyperkalemia, hyponatremia, and/or hypernatremia).

Gastrointestinal (GI) manifestations of COVID-19 include diarrhea, nausea, vomiting, abdominal pain, anorexia, anosmia, and dysgeusia.

Hepatobiliary (hepatic) manifestations of COVID-19 include abnormal levels of liver enzyme levels, including elevated aminotransferases (e.g. alanine aminotransferase and/or aspartate aminotransferase) and/or elevated bilirubin; and/or liver damage either caused directly by COVID-19 infection of liver cells or by drug hepatotoxicity, cytokine storm and/or pneumonia-associated hypoxia as secondary consequence of COVID-19 infection.

Endocrinologic manifestations of COVID-19 include hyperglycemia, ketoacidosis, diabetic ketoacidosis, euglycemic ketosis.

Neurologic and ophthalmologic manifestations of COVID-19 include mild neurological symptoms, such as headache, dizziness, myalgia and/or fatigue, anorexia, anosmia, and ageusia or more-severe manifestations, stroke (e.g. acute stroke, acute inflammatory demyelinating polyneuropathy (Guillain-Barre syndrome), and/or various forms of encephalopathy (e.g. meningoencephalopathy, hemorrhagic posterior reversible encephalopathy syndrome, or acute necrotizing encephalopathy). Particular ocular manifestations of COVID-19 include conjunctival congestion, conjunctivitis, eye redness, ocular irritation, foreign body sensation, tearing, and/or chemosis.

Dermatologic manifestations of COVID-19 include petechiae, livedo racemosa, livedo reticularis, erythematous rash, maculopapular rashes, urticaria, vesicles, pernio-like lesions, purpura and/or chilblains.

In a further preferred embodiment of the composition for use of the invention, the COVID-19 associated symptom is a fibrotic lung.

Fibrotic lungs as a result of the infection with SARS-CoV-2 are one of the most severe symptoms that often lead to a fatal outcome. Treatment options to alleviate these symptoms are presently severely limited. In accordance with the invention, patients suffering from a fibrotic lung will be preferably treated with transdermal patches, i.e. medicinal devices that contain the extract as an active ingredient. Methods for effecting the transport of actives through the skin are known in the art and will be implemented accordingly.

In WO2008/076229 A1 examples of transdermal formulations that can equally be applied for the compositions of the present invention are described and include but are not limited to, ointments, creams, gels, transdermal patches, sprays, lotions, mousses, aerosols, nasal sprays, buccal and sublingual tablets and tapes or adhesives, vaginal rings, and pastes. Transdermal administration can be accomplished by applying, pasting, rolling, attaching, pouring, pressing, rubbing, etc., of a transdermal preparation or formulation onto a skin or mucosal surface of a subject. The terms "transdermal delivery system," "transdermal patches" or simply "patches" refer to a polymeric matrix or liquid reservoir type of transdermal delivery device which is used to transdermally deliver defined doses of a substance, over a specific application period. Further guidance how such patches are prepared can be found in "Transdermal drug delivery" by Prausnitz MR, Langer R, Nat Biotechnol. 2008; 26(11):1261-8.

In a particularly preferred embodiment of the composition of the invention, namely when the affected organ is the lung, said composition is enriched with *Phyllanthus* derived lignans or gallotannins.

In a further preferred embodiment of the composition of the invention, the COVID-19 associated symptom is liver damage. In this case, the composition is preferably enriched with *Phyllanthus* derived lignans, gallotannins or flavonol-glycosides.

In an additional preferred embodiment of the composition of the invention, the COVID-19 symptom is damage of the heart, kidneys, or brain.

Also preferred is that the composition is administered in the form of a tablet or capsule; or in the form of a liquid as a spray, preferably a nasal spray or a mouth and throat spray. In one alternative, the liquid may be administered in vaporized form (nebulization) for example by inhalation. However, also envisaged herein is the administration as a dry powder e.g. by dry powder inhalation as commonly used for drug delivery in the treatment of various airway diseases.

In all embodiments of the invention, combinations of the various modes of administration including varying formulations are envisaged in case of need be. For example, a particularly preferred combined mode of administration is the administration in the form of a tablet (alternatively a capsule) and in the form of a mouth and throat spray, wherein it is understood that the tablet (alternatively capsule) and the mouth and throat spray may each be administrated concomitantly or at different time points within a certain period.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims.

The Examples illustrate the invention.

### Reference Example 1: Extraction of Phyllanthus amarus Leaves

Dried *Phyllanthus amarus* leaves were filled into an extraction device (steel vessel). 50% v/v EtOH was used as an extraction agent. Furthermore, disodiumhydrogen citrate at a final concentration of 0.1-1.0% mass/mass was added to the solution. The EtOH content was checked by measuring the density and corresponded to 35-45% mass/mass. The ratio of leaves to solvent was 1:10 (+/-3 solvent). The leaves were extracted for 1 hour at a temperature of between 30 and 50°C. Then, the mixture was washed with water over a filter (corresponds to three parts drugs) and pressed. Then, the mixture was filtered through a membrane having an exclusion volume of between 0.1 to 0.3 µm. Indian Sterculia gum, which had been dissolved in ethanol/water or in absolute ethanol before, was added to the solution. The mixture was then concentrated by means of evaporation under reduced pressure (about 300 mbar lowered to 20 mbar), a temperature of 30-60 °C. (+/-5 °C) being used, until the material had a dry content of 20 to 40% (mass/mass). Subsequently, the soft extract was mixed with maltodextrin until a homogeneous suspension was obtained. Then, the mixture was subjected to spray drying.

The values of the pesticides analysed herein are shown in Table 3, the ellagitannin values are shown in Table 4.

**Table 3**

| | **Pesticide value in the end product (in ppm)** | | | **Detection limit (in ppm)** |
|---|---|---|---|---|
| | **Ref. Example 1** | **Ref. Example 2** | **Ref. Example 3** | |
| **α-endosulfane** | **n.d** | **0.01** | **n.d.** | **0.01** |
| **β-endosulfane** | **<0.01** | **0.01** | **n.d.** | **0.01** |
| **endosulfane sulphate** | **<0.2** | **0.02** | **n.d.** | **0.02** |
| **a-HCH** | **<0.005** | **0.006** | **n.d.** | **0.005** |
| **lindane (y-HCH)** | **0.018** | **0.016** | **n.d.** | **0.005** |

| | | | | |
|---|---|---|---|---|
| **n.d.: not detectable** | | | | |

**Table 4**

| | **Values in the end product (in %)** | | |
|---|---|---|---|
| | **Ref. Example 1** | **Ref. Example 2** | **Ref. Example 3** |
| **ellagitannins** | **11.44** | **10.69** | **12.65** |

### Reference Example 2: Herbal preparation of Phyllanthus amarus Leaves

The method was carried out according to Reference Example 1 with the following modifications: After maltodextrin was added, the mixture was placed in a short-time heater at a temperature of 100°C for 36 seconds. Thus, the microbial contamination of the drug was reduced. The mixture was dried until the water content was below 5%. During the drying process, the temperature at the outlet of the heating unit did not exceed 90°C (+/-5%). Silica was added during and after the drying process.

The dried product was mixed and then sieved.

### Reference Example 3: Extraction of Phyllanthus amarus Leaves

The method was carried out according to Reference Example 1 with the following modifications: Prior to the filtration, a lipid (Miglyol) was added to the mixture (final concentration of 54.9% mass/mass relative to the extractive agents). Then, the mixture was filtered through a membrane having an exclusion volume of between 0.1 and 0.3 µm. Due to the previous addition of a lipid, the contaminations of lipophilic organic contaminations are removed during the ultra filtration. The subsequent concentration, mixing and spray drying have been described in Reference Example 1. The values of the pesticides analysed herein are shown in Table 3, the ellagitannin values are shown in Table 4. It can be seen from Table 3 that, compared to Reference Examples 1 and 2, the addition of a lipid prior to the filtration led to a significant removal of the undesired lipophilic contaminations and/or residues below the detection limit.

Table 4 shows clearly that the ellagitannins are not removed.

### Example 1: Toxicology of PTX-111

HFM, Inc. has completed acute toxicity and 30-day DRF studies for subchronic and chronic toxicity. According to ICH, EMA and FDA guidelines a complete toxicity program will be finalized.

### ACUTE TOXICITY

ORAL APPLICATION OF *PTX-111* IN TWO SPECIES ACCORDING TO EC GUIDELINES B.1.
NMRI-mice

| | |
|---|---|
| Tested dose level: | 5000 mg PTX-111 (= 2860 mg BDS*) /kg b.w. |
| No-effect dose level: | > 5000 mg/kg b.w. |

### DOSE LEVEL WITH FIRST

Intolerance reactions: > 5000 mg/kg b.w.
Lowest lethal dose level: > 5000 mg/kg b.w.
LD50: > 5000 mg/kg b.w.
Under the present test conditions a single oral administration of 5000 mg PTX-111/kg b.w. to mice revealed no signs of toxicity. The amount of 2860 mg
*Phyllanthus amarus* derived Botanical Drug Substance (BDS)/kg b.w. corresponded to 15.73 g dried *Phyllanthus amarus* leaves/kg b.w.

*BDS - Botanical Drug Substance
Sprague-Dawley rats
Tested dose level: 5000 mg PTX-111 (= 2860 mg BDS) / kg b.w.
No-effect dose level: > 5000 mg/kg b.w.

### DOSE LEVEL WITH FIRST

| | |
|---|---|
| Intolerance reactions: | > 5000 mg/kg b.w. |
| Lowest lethal dose level: | > 5000 mg/kg b.w. |
| LD50: | > 5000 mg/kg b.w. |

Under the present test conditions a single oral administration of 5000 mg PTX-111/kg b.w. to rats revealed no signs of toxicity. The amount of 2860 mg *Phyllanthus amarus* derived Botanical Drug Substance (BDS)/kg b.w. corresponds to 15.73 g dried *Phyllanthus amarus* leaves/kg b.w.

### INTRAPERITONEAL (I.P.) APPLICATION OF PTX-111 IN TWO SPECIES ACCORDING TO EC GUIDELINES B.1.

### NMRI-mice

| | |
|---|---|
| Tested dose levels: | 125 mg PTX-111 (= 71.5 mg BDS) / kg b.w. |
| | 250 mg PTX-111 (= 143.0 mg BDS) / kg b.w |
| | 500 mg PTX-111 (= 286.0 mg BDS) / kg b.w. |
| | 1000 mg PTX-111 (= 572.0 mg BDS) / kg b.w. |
| | 2000 mg PTX-111 (= 1144.0 mg BDS) / kg b.w. |
| No-effect dose level: | 125 mg/kg b.w. |

### Dose level with first

| | |
|---|---|
| Intolerance reactions: | 250 mg/kg b.w. |
| Lowest lethal dose level: | 500 mg/kg b.w. |

### LD50 (FINNEY; Probit Analysis)

| | | |
|---|---|---|
| at 24 hours: | ♂ | 1414 mg (= 809 mg BDS) / kg b.w. |
| | ♀ | 1040 mg (= 595 mg BDS) / kg b.w. |
| at 14 days: | ♂ | 520 mg (= 297 mg BDS) / kg b.w. |
| | ♀ | 684 mg (= 391 mg BDS) / kg b.w. |

Under the present test conditions a single intraperitoneal administration of 125, 250, 500, 1000 and 2000 mg PTX-111/kg b.w. to mice, the lowest lethal dose level was 500 mg PTX-111/kg b.w. This amount corresponds to 286 mg *Phyllanthus amarus* derived Botanical Drug Substance (BDS)/kg b.w. and corresponds to 1.57 g dried *Phyllanthus amarus* leaves/kg b.w.

### Sprague-Dawley rats

| | |
|---|---|
| Tested dose levels: | 500 mg PTX-111 (= 286 mg BDS) / kg b.w. |
| | 1000 mg PTX-111 (= 572 mg BDS) / kg b.w. |
| | 2000 mg PTX-111 (= 1144 mg BDS) / kg b.w. |
| No-effect dose level: | 500 mg/kg b.w. |

### Dose level with first

| | | |
|---|---|---|
| Intolerance reactions: 1000 mg/kg b.w. | | |
| Lowest lethal dose level: 1000 mg/kg b.w. LD50 FINNEY (Probit Analysis) | | |
| at 24 hours: | ♂ | 1012 mg (= 579 mg BDS)/kg b.w. |
| | ♀ | 1012 mg (= 579 mg BDS)/kg b.w. |
| at 14 days: | ♂ | 1012 mg (= 579 mg BDS)/kg b.w. |
| | ♀ | 1012 mg (= 579 mg BDS)/kg b.w. |

Under the present test conditions a single intraperitoneal administration of 500, 1000 and 2000 mg PTX-111/kg b.w. to rats, the lowest lethal dose level was 1000 mg PTX-111/kg b.w. This amount corresponds to 572 mg *Phyllanthus amarus* derived Botanical Drug Substance (BDS)/kg b.w. and corresponds to 3.15 g dried *Phyllanthus amarus* leaves/kg b.w.

### REPEATED DOSE TOXICITY

Oral application by gavage of *PTX-111* in two species for 30 days (DRF studies for 13-week subchronic toxicity studies).

### Sprague-Dawley rats

| | |
|---|---|
| Tested dose levels: | 300 mg PTX-111 (= 178.2 mg BDS) / kg b.w. |
| | 900 mg PTX-111 (= 534.7 mg BDS) / kg b.w. |
| | 2700 mg PTX-111 (= 1604.1 mg BDS) / kg b.w. |
| Mortality: | No mortality occurred |
| Body weight: | No substance-related influence was noted Food |
| consumption: | No substance-related influence was noted |

### Macroscopic post mortem findings: No changes were observed

Under the present test conditions, no signs of toxicity were observed in rats treated with either 300, 900 or 2700 mg PTX-111/kg b.w./day oral by gavage over a period of 30 days.

### Beagle dogs

### Tested dose levels:

| | |
|---|---|
| MTD phase | 300 mg PTX-111 (= 178.2 mg BDS) / kg b.w. |
| | 900 mg PTX-111 (= 534.7 mg BDS) / kg b.w. |
| | 2700 mg PTX-111 (= 1604.1 mg BDS) / kg b.w. |
| Fixed dose period | 900 mg PTX-111 (= 534.7 mg BDS) / kg b.w. |
| | 2700 mg PTX-111 (= 1604.1 mg BDS) / kg b.w. |

### Mortality:

| | |
|---|---|
| MTD phase | No mortality occurred |
| Fixed dose period | One of the female dogs died on test day 16 after treatment |
| | with 2700 mg PTX-111/kg b.w./day |

### Body weight:

| | |
|---|---|
| MTD phase | No substance-related influence was noted |
| Fixed dose period | No substance-related influence was noted |

### Food consumption:

| | |
|---|---|
| MTD phase | No substance-related influence was noted |
| Fixed dose period | No substance-related influence was noted |

### ECG:

| | |
|---|---|
| MTD phase | No substance-related abnormalities were noted in the animals treated with 2700 mg/kg b.w./day. No influence was noted on the heart rate. |

### Blood pressure:

| | |
|---|---|
| MTD phase | Blood pressure and respiratory rate were not affected by the treatment with 2700 mg/kg b.w./day |

### Haematology:

| | |
|---|---|
| Fixed dose period | None of the examined haematological parameters were influenced by the treatment with 900 or 2700 mg/kg b.w./day |

### Clinical biochemistry:

| | |
|---|---|
| Fixed dose period | aP, ALAT/GPT and ASAT/GOT was increased in the one |

Macroscopic post
surviving of the two female animals treated with 2700 mg/kg b.w./day. An effect was possibly already noted at the male animals of the 900 mg/kg b.w./day.

| | |
|---|---|
| mortem findings: | Macroscopic examination of prematurely on day 16 died female animal revealed a bronchial emphysema, an embolism in the aorta and a tightly filled gall-bladder |

Under the present test conditions, no toxic effects were observed in dogs treated with either 300 or 900 mg PTX-111/kg b.w./day oral by gavage in the MTD phase. Treatment with 2700 mg PTX-111/kg b.w./day corresponding to 1604.1 mg BDS/kg b.w./day for 30 days in the fixed dose period revealed once to several times repeated emesis, reduced motility, head shaking, tremor and discharge of faeces. One female dog died on test day 16.

### Example 2: Preparation of extract for pharmaceutical use

Whole uncut dry leaves are extracted in steel vessels with a drug solvent ratio 1:10 (± 3) of ethanol 50 % (V/V) for 1 to 3 hours at 30 - 40 °C. The drug residues are separated and then washed with 1 to 3 parts water. The liquid was subsequently filtrated through a membrane. Following the soft extract will be mixed with excipients until a homogenous suspension occurs. The mixture is processed through a short-heating unit for reduction of microbial contamination at a temperature of 80 to 120 °C. The mixture is thoroughly dried and subsequently sieved.

Quality: Identity and specification is evaluated by TLC or HPLC. The final extract contains 3 - 6 % lignan derivatives, calculated as hypophyllanthin and phyllanthin, determined by HPLC and a defined amount of gallotannins calculated as geraniin and corilagin determined by HPLC.

Biological tests (MAGI cell assay, measuring viral entry inhibition) with *PTX-111* are performed to ensure its antiviral activity in suitable antiviral test models. Microbial contamination, pesticide residues and heavy metals are again evaluated during batch analysis. Stability tests for the botanical drug substance and finished product have successfully been performed.

The finished product is a tablet containing 300 mg API, the native part of PTX-111. Details of the qualitative and quantitative composition of the trial medication are represented by Table 2.

**Table 2: Composition of the clinical trial medication**

| One sugar-coated tablet contains: | | |
|---|---|---|
| Component | Sugar-coated tablet amount (mg) | Specification |
| Tablet core: Active substance: | | |
| *Phyllanthus amarus* leaves dry extract Ethanolic 50 % (V/V) (3.5 - 7.5 : 1) | 300.000 | In house monograph |
| Excipients Extract Preparation: Total | 205.000 | |
| Sterculia | | BP |
| Maltodextrin | | USP 23 |
| Silica, colloidal anhydrous | | Ph Eur |
| Sodium acid citrate | | BP |
| Excipients tablet core : | | |
| Stearin-palmitin-acid | 6.000 | Ph Eur |
| Magnesium stearate | 6.000 | Ph Eur |
| Silicium-dioxid, dispersed | 9.000 | Ph Eur |
| Carbomethyl-starch-Na (Typ A) | 24.000 | Ph Eur |
| Cellulose, microcrystalline | 17.030 | Ph Eur |
| Cellulose, powdered | 33.000 | Ph Eur |
| | | |
| Nominal weight of tablet core: | 600.000 | |
| | | |
| Tablet Film-coat weight: | 360.000 | |
| Sugar-coat (sugars, gum, dyes) | 360.000 | Ph Eur/ AmFarbVo |
| Total Weight tablet: | 960.000 | |

Storage and Handling: *Sugar-coated tablets containing PTX-111* will be kept in a locked area with access to authorized persons under controlled room temperature conditions (between 15°C and 30°C).

### Example 3: In vitro testing of SARS-CoV-2 inhibitors

To establish an *in vitro* test system of SARS-CoV-2 inhibitors, kidney epithelial cells (Vero; ATCC CCL-81), human liver cells (HuH-7), and human lung carcinoma cells (A549) were infected with respiratory secretions of patients suffering from COVID-19. Virus stocks were obtained from a total of 10 different respiratory specimen, with viral titers peaking in Vero cells.

One of these strains (CA) induced cytopathic effects (CPE) in Vero cells and was further selected for testing, because viral replication and its inhibition can be monitored optically in addition to determination of viral loads in cell culture supernatants using quantitative realtime PCR (qPCR). The SARS-CoV-2 strain CA was expanded on Vero cells and stored in aliquots after filtration. The tissue culture infectious dose 50% (TCID₅₀) was determined using the method of Reed and Muench.

The inhibitory effect of different small molecules, peptides, and herbal extracts was evaluated using this experimental set-up. Vero cells were plated in 96-well flat bottom plates at 15,000 cells/well and cultivated in Dulbecco's Modified Eagle's Medium (DMEM), supplemented with 10% fetal calf serum, glutamine, and antibiotics. It is important to use the inner wells for testing only.

Cells were incubated with two- to fourfold dilutions of the inhibitory drugs prior to addition of the diluted SARS-CoV-2 strain CA, which was added at a multiplicity of infection (MOI) of 0.05. Each plate should include three wells of uninfected Vero cells and another three wells of Vero cells infected with strain CA in the absence of the inhibitor. To avoid measuring the input virus, media has to be changed 12-24 hours post plating. It is important to replenish the inhibitor at concentrations used before. The total volume should not exceed 150 µl per well.

The readout of the experiment is determination of the viral load in 50 µl cell cultures supernatant using an inhouse or commercial reverse transcriptase qPCR system. The Cobas 6800 system is appropriate to determine the viral loads; the Ct values can be converted into viral loads using defined standards. Subsequently, viral loads will be analyzed with respect to the dilutions of the inhibitor. We generate a sigmoidal curve using GraphPad Prism, which allows to calculate the effective concentration 50% (EC₅₀).

Many inhibitors inhibit viral replication by being cytotoxic. Thus, the experiment needs to control for cytotoxicity, which can be realized using the MTT lysis assay. After removal of the supernatant for viral load testing, 10 µl MTT reagent is added to each well. After formation of purple formazan crystals within 1-2 hours, detergent is added and the plate is incubated at 37°C overnight or at room temperature for more than one day. The absorbance at 595 nm can be measured using a photometer. The reduction of cell viability in the presence of the inhibitor needs to be compared in uninfected and infected cells and normalized to the viability in the absence of the inhibitor.

## Claims

1. A composition comprising or corresponding to a *Phyllanthus* extract for use in the treatment or prevention of a SARS-CoV-2 infection and/or at least one symptom of COVID-19.

2. The composition according to claim 1 for use according to claim 1, wherein the *Phyllanthus* extract is an extract of *Phyllanthus amarus* or *Phyllanthus niuri.*

3. The composition according to claim 1 or 2 for use according to claim 1 or 2, wherein the *Phyllanthus* extract is obtained or obtainable by a method comprising the following step(s):
(a) extracting *Phyllanthus* components with an ethanol/water mixture of 35-45% mass/mass which contains a heavy metal chelator in a concentration of 0.1-1% mass/mass; and optionally
(b) treating and concentrating the extract obtained in (a) with
(ba) Indian sterculia gum in a final concentration of 0.7 to 1.3% mass/mass, relative to the overall amount of extracted compounds; or
(bb) one or more pharmaceutically acceptable polysaccharides in a final concentration of 2-10% mass/mass, relative to the overall amount of extracted compounds; and/or optionally
(c) drying the extract obtained in step (a) or (b).

4. The composition according to claim 3 for use according to claim 3, wherein the *Phyllanthus* components are extracted from dried leaves.

5. The composition according to claim 3 or 4 for use according to claim 3 or 4, wherein after step (a) and prior to step (b) a filtration with the extract obtained in step (a) is carried out, wherein the filter preferably has an exclusion volume of 0.1-0.3 µm.

6. The composition according to any one of claims 3 to 5 for use according to any one of claims 3 to 5, wherein in step (a) or prior to step (b) a lipid, preferably selected from the group of plant oils, waxes and fatty acids is added in a final concentration of 1 - 100% mass/mass, relative to the overall amount of extracted compounds.

7. The composition according to any one of claims 1 to 6 for use according to any one of claims 1 to 6, which is a pharmaceutical composition or a medicinal device.

8. The composition according to claim 7 for use according to claim 7, which is to be administered orally, intravenously, topically and/or as an aerosol for the upper and lower respiratory tract.

9. The composition according to any one of claims 1 to 8 for use according to any one of claims 1 to 8, wherein the COVID-19-associated symptom is selected from
(a) pneumonia or severe pneumonia; or
(b) pulmonary fibrosis; or
(c) acute lung injury.

10. The composition according to any one of claims 1 to 8 for use according to any one of claims 1 to 8, wherein the COVID-19-associated symptom is a fibrotic lung, and wherein the composition is on, or embedded in, a transdermal patch.

11. The composition according to any one of claims 1 to 10 for use according to any one of claims 1 to 10, wherein the composition is enriched with preferably *Phyllanthus* derived lignans and/or gallotannins.

12. The composition according to any one of claims 1 to 8 for use according to any one of claims 1 to 8, wherein the at least one COVID-19 symptom is an extra-pulmonary manifestation selected from
(a) a hematologic and/or immune system-related manifestation; or
(b) a cardiovascular manifestation; or
(c) a renal manifestation; or
(d) a gastrointestinal manifestation; or
(e) a hepatobiliary manifestation; or
(f) a neurologic and/or ophthalmologic manifestation.

13. The composition according to any one of claims 1 to 8 and 12 for use according to any one of claims 1 to 8 and 12, wherein the COVID-19-associated symptom is liver damage.

14. The composition according to claim 13 for use according to claim 13, further enriched with preferably *Phyllanthus* derived lignans, gallotannins and/or flavonol-glycosides.
